Europäisches Patentamt

European Patent Office

Office européen des brevets

(11) Veröffentlichungsnummer: **0 311 749**
**A1**

(12) **EUROPÄISCHE PATENTANMELDUNG**

(21) Anmeldenummer: **88110436.8**

(22) Anmeldetag: **30.06.88**

(51) Int. Cl.4: **A61F 2/36**

(30) Priorität: **17.09.87 DE 8712578 U**

(43) Veröffentlichungstag der Anmeldung:
**19.04.89 Patentblatt 89/16**

(84) Benannte Vertragsstaaten:
**AT CH DE ES FR GB IT LI NL**

(71) Anmelder: **Howmedica GmbH**
**Professor-Küntscher-Str. 1-5**
**D-2314 Schönkirchen ü. Kiel(DE)**

(72) Erfinder: **Vécsei, Vilmos,Prof. Dr.**
**Höfergasse 18**
**A-1090 Wien/Österreich(AT)**
Erfinder: **Obersteiner, Karl**
**Zollstrasse 3**
**CH-9434 Au(CH)**

(74) Vertreter: **Dipl.-Ing. H. Hauck Dipl.-Ing. E.**
**Graalfs Dipl.-Ing. W. Wehnert Dr.-Ing. W.**
**Döring**
**Neuer Wall 41**
**D-2000 Hamburg 36(DE)**

(54) Oberschenkelteil einer Hüftgelenkendoprothese.

(57) Oberschenkelteil einer Hüftgelenkendoprothese mit einem Schaft (10), der ggf. über einen Kragen (11) in einen abgewinkelten Hals (12) übergeht, der mit einer Gelenkkugel verbunden oder verbindbar ist, wobei der obere bzw. proximale Bereich des Schafts als Fachwerk (14) geformt ist dergestalt, daß die Zug- und Druckkraftverteilung an die des natürlichen Knochens angenähert ist.

FIG.1

EP 0 311 749 A1

## Oberschenkelteil einer Hüftgelenkendoprothese

Die Neuerung bezieht sich auf ein Oberschenkelteil einer Hüftgelenkendoprothese nach dem Oberbegriff des Anspruchs 1.

Eine Schaftprothese hat bekanntlich die Aufgabe, den funktionsunfähigen proximalen Femur zu ersetzen, insbesondere den Femurkopf. Bei der Implantation werden Femurkopf und -hals reseziert; der Prothesenschaft wird in den freigebohrten Femurkanal eingeschlagen. Der Schaft wird entweder mit Knochenzement im Femurkanal verankert oder hält sich allein durch Kraftschluß (zementlose Prothese). Im letzteren Fall ist der Prothesenschaft verhältnismäßig lang ausgeführt und wird mit Oberflächenunebenheiten, Löchern oder dergleichen versehen, damit eine Verwachsung mit dem Knochen erfolgt.

Bei den Oberschenkelteilen von Hüftgelenkendoprothesen können zwei Komplikationen eintreten, die bisher nicht völlig beherrschbar sind. Die Belastung bei einem Femur ist im proximalen Bereich eine Zug-Druckbelastung. Im lateralen Bereich findet vorwiegend eine Zugbelastung statt, während im medialen Bereich Druckbelastung vorherrscht. Da die Schaftprothese dem Verlauf des proximalen Femurs angenähert ist, wird die Prothese in ähnlicher Weise belastet. Endoprothesen bestehen üblicherweise aus körperverträglichem Metall oder einer körperverträglichen Metallegierung. Die hierfür verwendeten Werkstoffe haben einen anderen Elastizitätsmodul als der natürliche Knochen, der im übrigen in Abhängigkeit von der jeweils auftretenden Belastung eine sehr unterschiedliche Innenstruktur aufweist. Die üblicherweise auftretende Belastung ist eine sogenannte Wechsellast, die zu ständiger, wenn auch relativ kleiner, Formänderungsarbeit im Knochen bzw. der Prothese führt. Diese Formänderungsarbeit verursacht bei der Prothese, vor allen Dingen im Übergangsbereich zwischen Prothese und angrenzender Knochenfläche, eine Lockerung, wodurch die Hüftgelenkendoprothese funktionsunfähig werden kann und ggf. entfernt werden muß. Eine Lockerung tritt auch bei der Verwendung von Knochenzement ein. Der Knochenzement selbst ist fest mit dem Knochen verbunden, während die Lockerung zwischen dem Zement und der Prothese, bzw. zwischen Zement und Knochen oder beiden stattfindet. Die Reoperation von Hüftgelenkendoprothesen ist eine sehr komplizierte und den Patienten stark belastende Prozedur.

Bei Schaftprothesen, falls nicht geschmiedet oder bei ungeeignetem Legierungsmaterial, kommt es auch immer wieder zu Schaftbrüchen. Manchmal beruhen sie auf nicht einwandfrei gegossenen Prothesenteilen, die Spannungsrisse oder Lunker enthalten können. Diese Erscheinung ist jedoch äußerst selten. Vielmehr wurde festgestellt, daß auch vollständig einwandfreie Prothesenschäfte brechen können, wenn sie über einen längeren Zeitraum implantiert waren. Es handelt sich um sogenannte Ermüdungsbrüche.

Der Neuerung liegt daher die Aufgabe zugrunde, ein Oberschenkelteil einer Hüftgelenkendoprothese zu schaffen, das sich im Hinblick auf die Aufnahme der Belastung annähernd so wie der lebende Knochen verhält. und vom Knochen voll inkorporiert wird.

Diese Aufgabe wird durch die Merkmale des Kennzeichnungsteils des Anspruchs 1 gelöst.

Bei der Neuerung ist von der Erkenntnis ausgegangen worden, daß es mit einer massiven Ausbildung des Schaftes nicht möglich ist, diesen so aufzubauen, daß er ein ähnliches Elastizitätsverhalten aufweist wie der natürliche Knochen. Daher sieht die Neuerung eine fachwerkartige Gestaltung des Schaftes im oberen oder proximalen Bereich vor. Der untere Schaft kann massiv geformt werden, da er normalerweise in bezug auf eine Bruchentstehung unkritisch ist, aber für die primäre Fixation der frisch implantierten Prothese eine Rolle spielt.

Ein aus Stegen bestehendes Fachwerk kann ausreichend steif gemacht werden, damit die auftretenden Belastungen der Höhe nach aufgenommen werden können und nicht zu einer plastischen Verformung führen. Entscheidend ist indessen, daß die Festigkeit und die Anzahl bzw. die Lage der Stäbe so gewählt werden können, daß das Festigkeits- bzw. Elastizitätsverhalten eines natürlichen Knochens im entsprechenden Bereich nachgebildet wird. Da die Lastverteilung im natürlichen Femur bekannt ist und auch dessen physikalischer Aufbau, ist es mit Hilfe eines Computers ohne weiteres möglich, das Fachwerk so zu berechnen und auszulegen, daß die gewünschten Eigenschaften erhalten werden.

Das Fachwerk des Schaftes muß naturgemäß so ausgeführt sein, daß es gleichwohl mehr oder weniger passend in den Femurkanal eingesetzt werden kann und hierbei auch nicht stört. Daher sieht eine Ausgestaltung der Neuerung vor,daß die Außenkontur im Fachwerkbereich durch in Längsrichtung des Schafts verlaufende Längsstäbe bestimmt wird,die durch Verbindungsstäbe gegeneinander abgestützt sind. Vorzugsweise sind die Längsstäbe so angeordnet, daß sie die vorzugsweise abgerundeten Kanten der seitlichen Begrenzungsflächen des Schaftes bilden. Vorder- und Rückseite eines Schaftes sind üblicherweise von annähernd parallelen Flächen gebildet, während die beiden anderen gegenüberliegenden Flächen im Querschnitt bogenförmig sind. Diese Kontur bleibt durch die Längsstäbe beibehalten, wobei jedoch zwischen den Längsstäben ein Hohlraum gebildet ist. Erste Verbindungsstäbe können benachbarte Längsstäbe miteinander verbinden. Ihre Außenflächen liegen daher in gleicher Ebene wie die zugeordnete Schaftaußenfläche. Zweite Verbindungsstreben können auch diagonal liegende Längsstreben miteinander verbinden.

Um Kerbwirkungen zu vermeiden, gehen die Längsstreben allmählich in den massiven distalen Bereich des Schaftes über. Daher sind gemäß einer weiteren Ausgestaltung der Neuerung im massiven Bereich in die Außenfläche des Schaftes furchenartige Vertiefungen geformt, die sich von der Außenfläche des Schaftes ausgehend zum proximalen Ende hin zunehmend vertiefen; sie lassen die Längsstreben beidseits der Vertiefungen allmählich aus dem massiven Schaftbereich hervortreten.

Die neuerungsgemäße Prothese besteht naturgemäß aus einem körperverträglichen Material. Sie wird vorzugsweise gegossen. Selbst relativ komplizierte Fachwerkstrukturen lassen sich gießtechnisch ohne weiteres bewältigen. Fertigungstechnisch können aber das Schmieden, das Sintern, und auch Kombinationsverfahren in Frage kommen.

Die Neuerung wird nachfolgend anhand eines Ausführungsbeispiels näher erläutert.

Fig. 1 zeigt die Rückseite der neuerungsgemäßen Endoprothese.

Fig. 2 zeigt die Vorderseite der Endoprothese nach Fig. 1.

Fig. 3 zeigt die mediale Seite der Endoprothese nach Fig.1 in Richtung Pfeil 3.

Fig. 4 zeigt die laterale Seite der Endoprothese nach Fig.1 in Richtung Pfeil 4.

Bevor auf die in den Zeichnungen dargestellten Einzelheiten näher eingegangen wird, sei vorangestellt, daß jedes der beschriebenen Merkmale für sich oder in Verbindung mit Merkmalen der Ansprüche von neuerungswesentlicher Bedeutung ist.

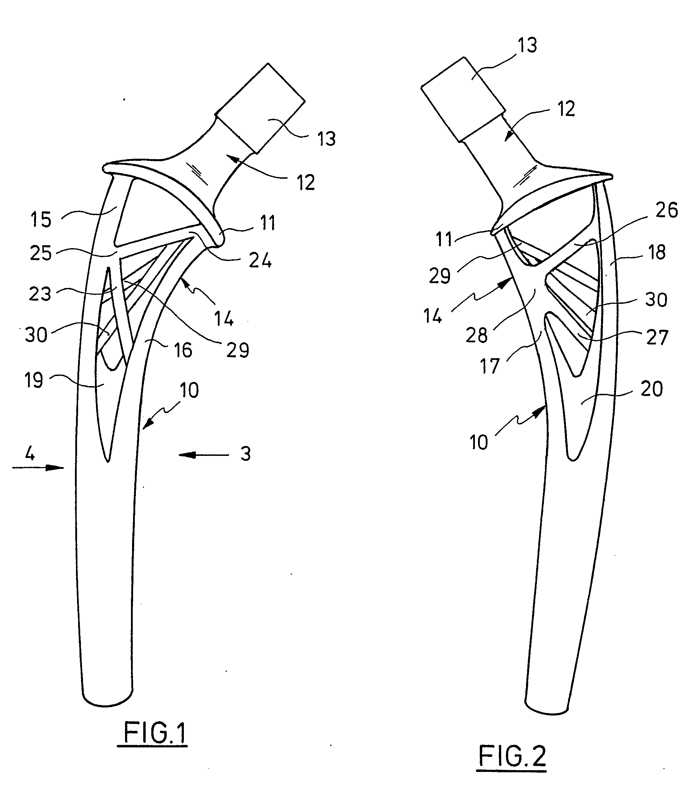

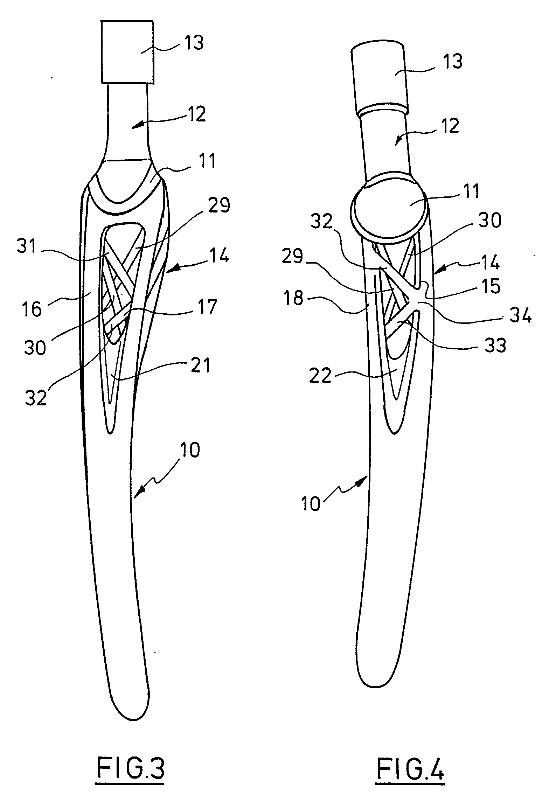

Der in den Figuren 1 bis 4 dargestellte Oberschenkelteil einer Hüftgelenkendoprothese weist einen Schaft 10 auf, der im proximalen Bereich durch einen Kragen 11 abgeschlossen ist. Der Kragen 11 steht nur medial und lateral etwas über. An den Kragen schließt sich ein Hals 12 an, an den ein Außenkonus 13 angeformt ist zur Aufnahme einer Gelenkkugel mit Innenkonus.

Vom distalen Ende des Schaftes 10 aufwärts sind annähernd zwei Drittel in herkömmlicher Weise massiv geformt. Das obere Drittel ist als Fachwerk 14 geformt. Das Fachwerk 14 weist vier Längsstäbe 15, 16, 17 und 18 auf. Die Längsstäbe 15 bis 18 bestimmen die Außenkontur des proximalen Bereichs des Schafts 10, wobei sie im Kantenbereich liegen zwischen den vier Seitenflächen des Schafts 10. Die äußeren Kanten der Stäbe 15 bis 18 sind abgerundet. Zwischen benachbarten Längsstäben 15 bis 18 furchenartige Vertiefungen 19, 20, 21 und 22 geformt, die vom massiven Teil ausgehend sich nach proximal zunehmend vertiefen und verbreitern. Sie ermöglichen das allmähliche "Herauswachsen" der Längsstäbe 15 bis 18 aus dem massiven Teil des Schafts 10.

Die Längsstäbe 15, 16 sind durch zwei Verbindungsstäbe 23, 24 miteinander verbunden. Die Verbindungsstäbe 23, 24 sind auf annähernd der halben Höhe des Längsstabs 15 gemeinsam bei 25 angebunden und erstrecken sich annähernd im rechten Winkel voneinander fort, um zu den Enden des Längsstabs 16 zu gehen. Die Außenseiten der Verbindungsstäbe 23, 24 liegen annähernd auf gleicher Höhe wie die Außenseiten der Längsstäbe 15, 16. In entsprechender Weise sind die Längsstäbe 17, 18 durch zwei Verbindungsstäbe 26, 27 miteinander verbunden. Sie beginnen an einem gemeinsamen Anbindungspunkt 28 etwa auf der halben Höhe des Längsstabs 17 und erstrecken sich annähernd im rechten Winkel zu den Endbereichen des Längsstabs 18. Der Verlauf der Verbindungsstäbe 23, 24 einerseits und 26, 27 andererseits ist entgegengesetzt. Es sei hierbei angemerkt, daß bei den Figuren 1 und 2 die Verbindungsstäbe auf der jeweils nicht einsehbaren Seite der Längsstäbe aus zeichendarstellerischen Gründen fortgelassen wurden.

Die Längsstäbe 15, 17 sind über einen diagonal verlaufenden zweiten Verbindungsstab 29 miteinander verbunden. Der zweite Verbindungsstab 29 ist etwas unterhalb der Anbindung 25 an den Längsstab 15 angebunden und erstreckt sich annähernd am oberen Ende des Längsstabs 17. Die Längsstäbe 16, 18 sind durch eine diagonal verlaufende zweite Verbindungsstab 30 miteinander verbunden. Er beginnt im unteren Bereich am Längsstab 18 und erstreckt sich zum oberen Ende des Längsstabs 16.

Die Längsstäbe 16, 17 sind ebenfalls über zwei Verbindungsstäbe 31, 32 miteinander verbunden. Sie beginnen nahe zusammenliegend etwa auf halber Höhe des Längsstabs 17 und erstrecken sich annähernd im rechten Winkel voneinander fort zum Längsstab 16. Entsprechend sind die Längsstäbe 18, 15 über zwei Verbindungsstäbe 32a, 33 miteinander verbunden. Sie beginnen an einem gemeinsamen Anbindungspunkt 34 an einem Längsstab 15 und erstrecken sich annähernd im rechten Winkel voneinander fort zum Längsstab 18.

Die Längsstäbe haben einen erheblich größeren Querschnitt als die Verbindungsstäbe. Die Anzahl der Verbindungsstäbe, ihr Querschnitt sowie ihre räumliche Anordnung und Anbindung an die Längsstäbe ist jedoch nicht beschränkt auf das in den Figuren 1 bis 4 gezeigte Beispiel. Sie können unterschiedlich abgewandelt werden. Im übrigen ist es möglich, die räumliche Anordnung der Gitterstruktur und die Querschnitte durch eine Computerberechnung so auszulegen, daß eine Lastverteilung erhalten wird, wie sie auch beim natürlichen Knochen auftritt.

Die gezeigte Prothese wird vorzugsweise zementlos implantiert, wobei Knochensubstanz über das Fachwerk in das Protheseninnere einwachsen kann. Dieser Bereich sollte während der Operation auch mit Spongiosa gefüllt werden.

**Ansprüche**

1. Oberschenkelteil einer Hüftgelenkendoprothese mit einem Schaft, der ggf. über einen Kragen in einen abgewinkelten Hals übergeht, der mit einer Gelenkkugel verbunden oder verbindbar ist, dadurch gekennzeichnet, daß der obere bzw. proximale Bereich des Schafts (10) als Fachwerk (14) geformt ist dergestalt, daß die Zug- und Druckkraftverteilung an die des natürlichen Knochens angenähert ist.

2. Oberschenkelteil nach Anspruch 1, dadurch gekennzeichnet, daß die Außenkontur im Fachwerkbereich (14) durch in Längsrichtung des Schafts (10) verlaufende Längsstäbe (15 bis 18) bestimmt wird, die durch Verbindungsstäbe gegeneinander abgestützt sind.

3. Oberschenkelteil nach Anspruch 2, dadurch gekennzeichnet, daß Verbindungsstäbe (29, 30) sich auch durch den Hohlraum erstrecken, der zwischen den Längsstäben (15 bis 18) aufgespannt ist.

4. Oberschenkelteil nach Anspruch 2 oder 3, dadurch gekennzeichnet, daß die Längsstäbe (15 bis 18) so angeordnet sind, daß sie die vorzugsweise abgerundeten Kanten der seitlichen Begrenzungsflächen des Schafts (10) bilden.

5. Oberschenkelteil nach Anspruch 2 und 4, dadurch gekennzeichnet, daß erste Verbindungsstäbe (23, 24; 26, 27; 31, 32; 33) jeweils benachbarte Länsstäbe (15 bis 18) verbinden.

6. Oberschenkelteil nach Anspruch 2 und 4 oder 5, dadurch gekennzeichnet, daß zweite Verbindungsstäbe (29, 30) sich überkreuzend diagonal angeordnete Längsstäbe miteinander verbinden.

7. Oberschenkelteil nach einem der Ansprüche 2 bis 6, dadurch gekennzeichnet, daß die Längsstäbe (15 bis 18) allmählich in den massiven Bereich des Schafts (10) übergehen.

8. Oberschenkelteil nach Anspruch 7, dadurch gekennzeichnet, daß im massiven Bereich in den vier Außenflächen des Schafts (10) furchenartige Vertiefungen (19, 20, 21, 22) geformt sind, die sich von der Außenfläche des Schafts (10) ausgehend zum proximalen Ende hin zunehmend vertiefen und verbreitern und die Längsstäbe (15 bis 18) beidseits der Vertiefungen allmählich aus dem massiven Schaftbereich hervortreten.

9. Oberschenkelteil nach einem der Ansprüche 1 bis 8, dadurch gekennzeichnet, daß sie als Gußteil, Schmiedeteil oder in gesinterter Fertigung aus einer körperverträglichen Metallegierung oder einem entsprechenden Metall geformt sind.

10. Oberschenkelteil nach einem der Ansprüche 1 bis 9, dadurch gekennzeichnet, daß es mit einem Überzug aus Hydroxylapatit oder einem ähnlichen inerten Material versehen ist, das das Einwachsen von Knochensubstanz befördert.

FIG.1

FIG.2

FIG.3

FIG.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-3 536 894 (CESKE VYSOKE UCENI TECHNICKE V PRAZE) * Ansprüche 1, 4; Figur 1 * | 1 | A 61 F 2/36 |
| A | DE-A-3 028 393 (KOUTROLIKOS) * Ansprüche 1,2 * | 1 | |
| A | DE-A-2 941 266 (EB TORNIER) * Ansprüche 1, 2; Figur 1 * | 1 | |
| P,A | EP-A-0 245 846 (COPF) * Anspruch 1; Figur 1 * | 1 | |
| P,X | DE-U-8 712 578 (HOWMEDICA) * ganzes Dokument * | 1-10 | |

**RECHERCHIERTE SACHGEBIETE (Int. Cl.4)**

A 61 F 2/00

Der vorliegende Recherchenbericht wurde für alle Patentansprüche erstellt

| Recherchenort | Abschlußdatum der Recherche | Prüfer |
|---|---|---|
| BERLIN | 20-12-1988 | KANAL P K |